# EUROPEAN PATENT APPLICATION

(11) **EP 4 717 708 A1**
(43) Date of publication of application: **01.04.2026**
(21) Application number: 24811046.2
(22) Date of filing: 17.05.2024
(51) Int. Cl.: C07K 14/78, A61K 38/39, A61K 47/42, A61L 27/24, A61L 27/52, A61P 7/04, A61P 27/02, C07K 1/113, C08J 3/24, C08J 3/075, C12M 1/00, C12N 5/00

(54) **HYDROGEL COMPRISING COLLAGEN CROSSLINKED BY PLATINUM COMPLEX**

(30) Priority: 23.05.2023 JP 2023084525
(71) Applicant: WASEDA UNIVERSITY, Shinjuku-ku Tokyo 169-8050 (JP); The Kitasato Institute, Tokyo 108-8641 (JP)
(72) Inventor: KOIDE Takaki, Tokyo 169-8050 (JP); ICHISE Shinichiro, Tokyo 108-8641 (JP)
(74) Representative: Peters, Hajo
(86) International application number: PCT/JP2024/018273
(87) International publication number: WO 2024/242039

(57) **Abstract**

The purpose is to develop a crosslinked collagen hydrogel that can be easily manufactured and has high transparency, high safety, and low toxicity. It was found that a platinum-crosslinked collagen hydrogel that is colorless, transparent, and free of cytotoxicity can be manufactured by incubating collagen of a specific concentration and a platinum(II) complex of a specific concentration under conditions of a specific pH, and the present invention was accomplished. Specifically, the present invention provides a hydrogel that is composed of platinum-crosslinked collagen in which collagen is crosslinked by a platinum(II) complex and exhibits a visible light transmittance of 60% or more when it is measured at an absorbance of 400 nm in a cell having an optical path length of 10 mm.

## Description

### Technical Field

The present invention relates to a hydrogel composed of collagen crosslinked by a platinum complex and its use and manufacturing method.

### Background Art

New hydrogels applicable for medical applications, particularly in ophthalmology, have been studied (Patent documents 1 and 2).

Collagen is one of the proteins that typically constitute the dermis, ligaments, tendons, bones, cartilage, and so on of vertebrates, as collagen fibers and is a major component of extracellular substrate (extracellular matrices) of multicellular animals. A collagen molecule has a triple helix structure that is formed by three polypeptide chains (α chains) intertwined in a helical fashion.

When collagen is not crosslinked, a cloudy fibrous collagen gel is formed (see FIG. 1). Alternatively, a collagen gel is produced by crosslinking collagen with a crosslinking agent, which is being studied for the purpose of application as a medical material or the like (Patent documents 3 to 5). However, the collagen gels described in Patent documents 3 to 5, formed by using crosslinking agents such as glutaraldehyde, carbodiimide, genipin, or a photocrosslinker, have limitations on their use in the human body due to issues such as toxicity and have a problem of difficulty in use in vivo.

In contrast, it has been reported that dissolution of a platinum complex, cisplatin, in dimethyl sulfoxide (DMSO) causes ligand exchange to form [Pt(NH₃)₂(Cl)(DMSO)]⁺, which prevents formation of collagen fibers and suppresses cloudiness due to fibrosis (Non patent document 1). In addition, it has been demonstrated that a ligand-substituted platinum complex has lower toxicity than the one of cisplatin (Non patent documents 1 to 3). However, it has not been known that [Pt(NH₃)₂(Cl)(DMSO)]⁺ crosslinks collagen to form a hydrogel composed of crosslinked collagen.

### PRIOR ART DOCUMENTS

### Non patent documents

Non patent document 1: Zenda M. et al., Chem. Biol. Drug Des., 2015, 85: 519-526
Non patent document 2: Fischer S. J. et al., Neurotoxicology, 2008, 29: 444-452
Non patent document 3: Raghavan R. et al., Indian J. Pharmacol., 2015, 47: 322-324

### Patent documents

Patent document 1: JP-A-2017-121307
Patent document 2: JP-A-2020-150846
Patent document 3: International Publication No. WO 2005/079879
Patent document 4: JP-A-2014-103985
Patent document 5: International Publication No. WO 2008/147867

### Summary of Invention

### Technical Problem

It is an object of the present invention to develop a crosslinked collagen hydrogel that can be easily manufactured and has high transparency, high safety, and low toxicity.

### Solution to Problem

The present inventors intensively studied and discovered that collagen of a specific concentration and a platinum(II) complex of a specific concentration may be incubated under a specific pH condition to produce a platinum-crosslinked collagen hydrogel with high transparency and low cytotoxicity, thus completing the present invention. Furthermore, it was clarified that excess crosslinking agent used during the manufacturing process of the platinum-crosslinked collagen hydrogel can be effectively removed by dialysis or similar methods.

More specifically, the present invention provides a hydrogel that is composed of platinum-crosslinked collagen in which collagen is crosslinked by a platinum(II) complex, and exhibits a visible light transmittance of 60% or more when it is measured at an absorbance of 400 nm in a cell with a light path length of 10 mm.

The hydrogel of the present invention is composed of platinum-crosslinked collagen in which the collagen is crosslinked by a platinum(II) complex and may have a visible light transmittance of 60% or more when it is measured at an absorbance of 380 to 780 nm in a cell with a light path length of 10 mm.

The hydrogel of the present invention may be a hydrogel in which the platinum(II) complex includes dimethyl sulfoxide (DMSO) as a ligand.

In the hydrogel of the present invention, the platinum(II) complex may be a complex in which at least one ligand of cis-diamminedichloroplatinum(II) (hereinafter, also referred to as "cisplatin") and/or trans-diamminedichloroplatinum(II) (hereinafter, also referred to as "transplatin") is substituted with DMSO, that is, the platinum(II) complex may be a complex in which at least one ligand of a chloride ion and/or an ammine of cis-diamminedichloroplatinum(II) and/or trans-diamminedichloroplatinum(II) is substituted with DMSO, and more preferably may be a complex in which at least one chloride ion is substituted with DMSO. A particularly preferable platinum(II) complex is cis-type or trans-type [Pt(NH₃)₂(Cl)(DMSO)]⁺.

In the hydrogel of the present invention, the collagen may be atelocollagen.

The hydrogel of the present invention may be a hydrogel for use as an artificial vitreous body, a hemostatic agent, a sustained-release drug carrier, a tissue replacement material, or a scaffold material for cell culture.

In addition, the present invention provides a material for eye disease treatment composed of the hydrogel.

In addition, the present invention provides an artificial vitreous body composed of the hydrogel.

In addition, the present invention provides a hemostatic agent composed of the hydrogel.

In addition, the present invention provides a scaffold material for cell culture composed of the hydrogel.

In addition, the present invention provides a method for manufacturing a hydrogel, including a step of mixing and incubating collagen and a platinum(II) complex.

The method for manufacturing a hydrogel of the present invention may be a manufacturing method including a step of incubating the collagen having a concentration in a range of 1.0 to 4.0 mg/mL and the platinum(II) complex having a concentration in a range of 25 µM to 1 mM under conditions of a pH in a range of 5.0 to 8.0.

In addition, the present invention provides a method for manufacturing the hydrogel, comprising:
(a) a step of incubating collagen having a concentration in a range of 1.0 to 4.0 mg/mL and a platinum(II) complex having a concentration in a range of 25 µM to 1 mM under conditions of a pH less than 5.0; and
(b) a step of changing the pH condition to exhibit a pH of 5.0 to 8.0 to induce gelation.

The manufacturing method may include, between the steps (a) and (b), (a') a step of performing dialysis under conditions of a pH less than 5.0 to remove free platinum(II) complex.

### Advantageous Effects of Invention

The present invention can provide a hydrogel composed of platinum-crosslinked collagen that can be easily manufactured and has low toxicity and high transparency.

The hydrogel of the present invention has characteristics in which:
- the transparency is high;
- gel formation can be controlled by pH;
- the gel strength can be controlled by the concentration of a platinum(II) complex;
- gelation is reversible and the gel can be dissolved; and
- the unreacted crosslinking agent can be removed.

Consequently, the following advantages can be achieved:
(1) cell observation is easy and good data can be efficiently obtained even in a thick 3D culture;
(2) after being injected into a living body, such as the eyeball, and undergoing gelation, the gel can be dissolved and removed;
(3) the hardness of the gel can be adjusted according to the purpose, and it is therefore expected to be able to adjust the biodegradability; and
(4) the unreacted crosslinking agent does not cause any toxicity.

### Brief Description of Drawings

[FIG. 1] FIG. 1 shows schematic views of fibrous collagen (bottom left of the figure) and platinum-crosslinked collagen crosslinked by a cisplatin-DMSO complex (bottom right of the figure). The cisplatin-DMSO complex crosslinks collagen to prevent fibrosis of collagen, resulting in no formation of a cloudy hydrogel (fibrous collagen gel).
[FIG. 2] FIG. 2 shows the results of evaluation of gelation ability of platinum-crosslinked collagen formed when mixture solutions of atelocollagen and a cisplatin-DMSO complex of various concentrations were incubated in a buffer solution of pH 7.4 at 37°C for 30 minutes. The arrowheads in the photograph indicate stainless steel balls.
[FIG. 3] FIG. 3 is a graph of transmittance of light of 200 to 900 nm of a fibrous atelocollagen hydrogel (solid line) and a platinum-crosslinked atelocollagen hydrogel crosslinked by a cisplatin-DMSO complex (broken line).
[FIG. 4] FIG. 4 shows the results of evaluation of fibrous atelocollagen hydrogel-forming ability (upper row) and platinum-crosslinked atelocollagen hydrogel-forming ability (lower row) when atelocollagen of various concentrations (upper row) and mixture solutions (lower row) of atelocollagen of various concentrations and a cisplatin-DMSO complex were incubated in a buffer solution of pH 7.4 at 37°C for 30 minutes. The arrowheads in the photographs indicate stainless steel balls.
[FIG. 5] FIG. 5 shows the results of evaluation of fibrous atelocollagen hydrogel-forming ability (upper row) and platinum-crosslinked atelocollagen hydrogel-forming ability when atelocollagen (upper row) and a mixture solution (lower row) of atelocollagen and a cisplatin-DMSO complex were incubated at 37°C for 30 minutes under various pH conditions. The arrowheads in the photographs indicate stainless steel balls.
[FIG. 6] FIG. 6 shows the results of evaluation of platinum-crosslinked atelocollagen hydrogel-forming ability when a mixture solution of atelocollagen and a cisplatin-DMSO complex was incubated in a buffer solution of pH 7.4 at 37°C for 30 minutes under various ionic strength environments. The arrowheads in the photograph indicate stainless steel balls.
[FIG. 7] FIG. 7 shows the results of study on gelation time when a mixture solution of atelocollagen and a cisplatin-DMSO complex was incubated in a buffer solution of pH 7.4 at 37°C for 5 to 20 minutes. The arrowheads in the photograph indicate stainless steel balls.
[FIG. 8] FIG. 8 shows the results of study on influence of temperature on the gelation ability when a mixture solution of atelocollagen and a cisplatin-DMSO complex was incubated in a buffer solution of pH 7.4 at 4°C to 37°C for 30 minutes. The arrowheads in the photograph indicate stainless steel balls.
[FIG. 9] FIG. 9 shows the results of evaluation of gelation ability when a platinum-crosslinked atelocollagen solution obtained by dialysis of a mixture solution of atelocollagen and a cisplatin-DMSO complex in a 1 mM HCl aqueous solution at 4°C overnight and a solution obtained by changing the pH of the platinum-crosslinked atelocollagen solution to 7.4 were incubated at 37°C for 30 minutes. FIG. 9A shows the state before pH adjustment, and FIG. 9B shows the state after pH adjustment. The arrowheads in the photograph indicate stainless steel balls.
[FIG. 10] FIG. 10 shows the results of evaluation of gelation ability when mixture solutions of atelocollagen and a transplatin-DMSO complex of various concentrations were incubated in a buffer solution of pH 7.4 at 37°C for 30 minutes. The arrowheads in the photograph indicate stainless steel balls.
[FIG. 11] FIG. 11 shows the results of evaluation of platinum-crosslinked atelocollagen-forming ability when mixture solutions of atelocollagen of various concentrations and a transplatin-DMSO complex were incubated in a buffer solution of pH 7.4 at 37°C for 30 minutes. The arrowheads in the photograph indicate stainless steel balls.
[FIG. 12] FIG. 12 shows the results of evaluation of platinum-crosslinked atelocollagen hydrogel-forming ability when a mixture solution of atelocollagen and a transplatin-DMSO complex was incubated at 37°C for 30 minutes under various pH conditions. The arrowheads in the photograph indicate stainless steel balls.
[FIG. 13] FIG. 13 shows the results of evaluation of platinum-crosslinked collagen hydrogel-forming ability when a mixture solution of atelocollagen and a transplatin-DMSO complex was incubated in a buffer solution of pH 7.4 at 37°C for 30 minutes under various ionic strength environments. The arrowheads in the photograph indicate stainless steel balls.
[FIG. 14] FIG. 14 shows the results of study on hydrogel formation time when a mixture solution of atelocollagen and a transplatin-DMSO complex was incubated in a buffer solution of pH 7.4 at 37°C for 5 to 15 minutes. The arrowheads in the photograph indicate stainless steel balls.
[FIG. 15] FIG. 15 shows the results of study on temperature dependency of gelation when a mixture solution of atelocollagen and a transplatin-DMSO complex was incubated in a buffer solution of pH 7.4 at 4°C to 37°C for 30 minutes. The arrowheads in the photograph indicate stainless steel balls.
[FIG. 16] FIG. 16 shows the results of evaluation of heat stability when a fibrous atelocollagen hydrogel and a platinum-crosslinked atelocollagen hydrogel crosslinked by a cisplatin-DMSO complex were heated. The arrowheads in the photographs indicate stainless steel balls.
[FIG. 17] FIG. 17 shows the results of study on heat shrinkability after heating of a fibrous atelocollagen hydrogel and a platinum-crosslinked atelocollagen hydrogel crosslinked by a cisplatin-DMSO complex. FIG. 17A shows the state of a fibrous atelocollagen gel, FIG. 17B shows the state of a crosslinked atelocollagen gel before heating, and FIG. 17C shows the state of the crosslinked atelocollagen gel after heating for 30 minutes.
[FIG. 18] FIG. 18 shows phase contrast microscopic images and fluorescence microscopic images of HT-1080 cells cultured on a fibrous atelocollagen hydrogel or a platinum-crosslinked atelocollagen hydrogel crosslinked by a cisplatin-DMSO complex for 1.5 to 67 hours and stained with Hoechst 33342 and calcein-AM.
[FIG. 19] FIG. 19 is a graph showing the evaluation of cell proliferation activity by counting the number of cells cultured on a fibrous atelocollagen hydrogel or a platinum-crosslinked atelocollagen hydrogel crosslinked by a cisplatin-DMSO complex for 1.5 to 67 hours.
[FIG. 20] FIG. 20 shows microscopic images of gel slices of HT-1080 cells seeded on a fibrous atelocollagen hydrogel or a platinum-crosslinked atelocollagen hydrogel crosslinked by a cisplatin-DMSO complex and cultured for 4 days and stained with hematoxylin and eosin (HE). FIG. 20A shows a microscopic image of a fibrous atelocollagen hydrogel, and FIG. 20B shows a microscopic image of a platinum-crosslinked atelocollagen hydrogel.
[FIG. 21] FIG. 21 shows confocal fluorescence microscopic images of 3-dimensional cell clusters obtained when MDCK cells derived from a canine renal tubular cell line were mixed and embedded in a fibrous gel (FIG. 21A) and a platinum(II) complex-crosslinked gel (FIG. 21B) and cultured for 6 days. FIG. 21C shows a relationship between the depth of visual field and the fluorescence intensity when MDCK cells derived from a canine renal tubular cell line were mixed and embedded in a fibrous gel (denoted as FG) and a platinum(II) complex-crosslinked gel (denoted as PCG) and cultured for 6 days.
[FIG. 22] FIG. 22 shows the results of study on gel dissolution of a platinum(II) complex-crosslinked gel by addition of reduced glutathione.
[FIG. 23] FIG. 23 shows the results of study on the addition amount of a platinum(II) complex and the hardness adjustment of a gel. FIG. 23A shows the degree of deformation of a 50 µM transplatin-DMSO crosslinked collagen gel when pressed down from above with a spatula, FIG. 23B shows that of a 100 µM transplatin-DMSO crosslinked collagen gel, FIG. 23C shows that of a 300 µM transplatin-DMSO crosslinked collagen gel, FIG. 23D shows that of a 500 µM transplatin-DMSO crosslinked collagen gel, and FIG. 23E shows that of a 1 mM transplatin-DMSO crosslinked collagen.
[FIG. 24] FIG. 24 shows the results when atelocollagen gels containing transplatin-DMSO of various concentrations from 50 to 800 µM were prepared and the elastic moduli thereof were measured with a universal tester.

### Description of Embodiments

### 1. Definition

In the present specification, the term "platinum(II) complex" refers to a platinum complex having a function of crosslinking collagen. Specifically, the platinum(II) complex refers to a complex of which the ligand is dimethyl sulfoxide (DMSO). More specifically, the platinum(II) complex refers to a complex in which at least one ligand of cis-diamminedichloroplatinum (II) (hereinafter, also referred to as "cisplatin") is substituted with DMSO, more preferably at least one chloride ion and/or ammine of cisplatin is substituted with DMSO (in the present specification, also referred to as "cisplatin-DMSO complex") or at least one ligand of trans-diamminedichloroplatinum (II) (hereinafter, also referred to as "transplatin") is substituted with DMSO, and more preferably at least one chloride ion and/or ammine of transplatin is substituted with DMSO (in the present specification, also referred to as "transplatin-DMSO complex"). Furthermore, specifically, the "cisplatin-DMSO complex" includes cis-type [Pt(NH₃)₂(Cl)(DMSO)]⁺ that is generated by dissolving a platinum complex, cisplatin, in dimethyl sulfoxide (DMSO), and "transplatin-DMSO complex" includes trans-type [Pt(NH₃)₂(Cl)(DMSO)]⁺ that is generated by dissolving transplatin in dimethyl sulfoxide (DMSO).

In the present specification, the term "platinum-crosslinked collagen" refers to a collagen derivative that is obtained by crosslinking collagen by the "platinum(II) complex" (see FIG. 1).

In the present specification, the "platinum-crosslinked collagen hydrogel" refers to a hydrogel that is obtained by crosslinking collagen by the platinum(II) complex, more specifically, refers to a hydrogel that is obtained by placing an aqueous solution containing platinum-crosslinked collagen obtained by crosslinking collagen by the platinum(II) complex under conditions of pH 5.0 to 8.0.

In the present specification, the term "composed of" is used to mean "being formed of" and is distinguished from "consisting of' to limit the components.

### 2. Hydrogel composed of platinum-crosslinked collagen

One embodiment of the present invention is a hydrogel composed of platinum-crosslinked collagen (also referred to as "platinum-crosslinked collagen hydrogel").

The collagen gel of the present invention is composed of platinum-crosslinked collagen in which collagen is crosslinked by a platinum(II) complex and exhibits a visible light transmittance of 60% or more when it is measured at an absorbance of 400 nm, preferably 380 to 780 nm, in a cell with a light path length of 10 mm, and provides a hydrogel having high transparency in the visible light region.

The platinum-crosslinked collagen hydrogel of the present invention can be manufactured by a manufacturing method including a step of incubating collagen of a specific concentration and a platinum(II) complex of a specific concentration under a specific pH condition. Preferable manufacturing conditions are that, as shown in Examples described later, the collagen concentration is preferably in a range of 1.0 to 4.0 mg/mL, more preferably in a range of 1.5 to 4.0 mg/mL, and further preferably in a range of 1.5 to 3.5 mg/mL, the concentration of the platinum(II) complex is preferably in a range of 25 to 200 µM, more preferably in a range of 50 to 200 µM, further preferably in a range of 50 to 150 µM, and most preferably in a range of 100 to 200 µM, and the pH is preferably 5.0 to 8.0, more preferably in a range of 6.0 to 8.0, and further preferably in a range of 7.0 to 8.0, and pH 7.4 is particularly preferable.

The manufacturing of a platinum-crosslinked collagen hydrogel of the present invention can use collagen as a raw material, preferably, can use fiber-forming collagen. The fiber-forming collagen is known to have several types including Type I and Type II, and any of these types can be used in the manufacturing of the hydrogel of the present invention. From the viewpoint of high gelation ability, Type I collagen is particularly preferable. In addition, in the manufacturing of the hydrogel of the present invention, collagen derived from vertebrates, such as a cow, a horse, a pig, a rabbit, a mouse, a monkey, a human, a rainbow trout, a tilapia, and a catfish, can be used.

Generally, a non-triple helix region (telopeptide) is present at the end of a collagen triple helix and is a main antigen site of the collagen. It is known that the antigenicity of collagen is significantly decreased by removing the telopeptide by enzyme treatment, and the collagen from which the telopeptide is removed by enzyme treatment is called atelocollagen. In the manufacturing of the hydrogel of the present invention, atelocollagen can be suitably used.

Commercially available collagen and atelocollagen can be used in the manufacturing.

The platinum(II) complex for use in manufacturing the platinum-crosslinked collagen may be either one or both of a cisplatin-DMSO complex and a transplatin-DMSO complex.

As shown in Examples below, the hydrogel of the present invention has characteristics in which:
- the transparency is high;
- gel formation can be controlled by pH;
- the gel strength can be controlled by the concentration of a platinum(II) complex;
- gelation is reversible and the gel can be dissolved; and
- the unreacted crosslinking agent can be removed.

Consequently, the following advantages are achieved:
(1) cell observation is easy and good data can be efficiently obtained even in a thick 3D culture;
(2) after being injected into a living body, such as the eyeball, and undergoing gelation, the gel can be dissolved and removed;
(3) the hardness of the gel can be adjusted according to the purpose, and it is therefore expected to be able to adjust the biodegradability; and
(4) the unreacted crosslinking agent does not cause any toxicity.

The platinum-crosslinked collagen hydrogel of the present invention has high transparency, does not show cytotoxicity, and exhibits high safety and therefore can be used as a material for treatment of an eye disease, such as macular hole, retinal detachment, and retinal tear, an artificial vitreous body, a hemostatic agent (preferably a tamponade material), a sustained-release drug carrier, a tissue replacement material, or a scaffold material for cell culture. In particular, since the free platinum(II) complex can be removed by dialysis after crosslinking collagen by a platinum(II) complex and before the generation of a hydrogel by the platinum-crosslinked collagen, the platinum-crosslinked collagen hydrogel of the present invention can suppress cytotoxicity resulting from a crosslinking agent and can be used safely not only in cell culture in vitro but also as a material for treatment in vivo of an eye disease or the like. Furthermore, since the platinum-crosslinked collagen of the present invention can appropriately control the induction of gelation by adjusting, for example, the pH condition, it is possible to gelate in vivo a platinum-crosslinked collagen solution in a sol state injected into the body. Accordingly, it is possible to provide a material for treatment that can reduce the burden on subjects receiving it when applied to the body. The platinum-crosslinked collagen of the present invention, as shown in Examples below, can control the gel strength by the concentration of the platinum(II) complex, and the gelation is reversible, and it is possible to dissolve the gel.

### 3. Method for manufacturing platinum-crosslinked collagen hydrogel

Another embodiment of the present invention is a method for manufacturing a platinum-crosslinked collagen hydrogel.

The method for manufacturing a platinum-crosslinked collagen hydrogel is, as described above, a manufacturing method including a step of incubating collagen of a specific concentration and a platinum(II) complex of a specific concentration under a specific pH condition.

In the method for manufacturing a platinum-crosslinked collagen hydrogel, preferably, the concentration of the collagen is in a range of 1.0 to 4.0 mg/mL, the concentration of the platinum(II) complex is in a range of 25 to 200 µM, and the pH is in a range of 5.0 to 8.0. The concentration of the collagen is more preferably 1.5 to 4.0 mg/mL and further preferably in a range of 1.5 to 3.5 mg/mL. The concentration of the platinum(II) complex is more preferably in a range of 50 to 200 µM, further preferably in a range of 50 to 150 µM, and the most preferably in a range of 100 to 200 µM. The pH is more preferably in a range of 6.0 to 8.0 and further preferably in a range of 7.0 to 8.0, and pH 7.4 is particularly preferable.

The collagen as a raw material of the platinum-crosslinked collagen hydrogel is preferably atelocollagen, and a commercially available product can be used.

The platinum(II) complex may be either one or both of a cisplatin-DMSO complex and a transplatin-DMSO complex.

The method for manufacturing the hydrogel may be a manufacturing method comprising:
(a) a step of incubating collagen having a concentration in a range of 1.0 to 4.0 mg/mL and a platinum(II) complex having a concentration in a range of 25 to 200 µM under conditions of a pH less than 5.0; and
(b) a step of changing the pH condition to exhibit a pH of 5.0 to 8.0 to induce gelation.

The pH condition in the step (a) is more preferably a pH of greater than 1.0 and less than 5.0 and further preferably in a range of 2.0 to 4.0, and pH 3.0 is particularly preferable.

The pH condition in the step (b) is more preferably a range of 6.0 to 8.0 and further preferably a range of 7.0 to 8.0, and pH 7.4 is particularly preferable.

Furthermore, the manufacturing method may include, between the steps (a) and (b), (a') a step of removing free platinum(II) complex by dialysis under conditions of a pH less than 5.0.

The pH condition of the step (a') may be the same as or different from that in the step (a). The pH condition in the step (a') is more preferably a pH of greater than 1.0 and less than 5.0 and further preferably a pH in a range of 2.0 to 4.0, and pH 3.0 is particularly preferable.

### EXAMPLES

The present invention will be described further specifically with reference to Examples and Comparative Examples below but can be appropriately modified without departing from the gist of the present invention. Accordingly, the scope of the present invention is not to be construed as being limited to the specific examples shown below.

### I. Materials and method

### 1. Gelation ability evaluation (1)

Atelocollagen (final concentration: 2.5 mg/mL) and a cisplatin-DMSO complex solution of various concentrations (final concentration: 0 to 200 µM) were mixed, and an atelocollagen/cisplatin-DMSO complex mixture solution containing a physiological phosphate buffer solution [30 mM phosphate buffer solution (pH 7.4), 120 mM NaCl] was prepared and incubated at 37°C for 30 minutes. Whether a hydrogel was formed or not was evaluated using whether a stainless steel ball with a diameter of 1.5 mm and a specific gravity of 7.8 g/cm³ was held on the interface or not as an indicator. The results are shown in FIG. 2.

### 2. Transparency evaluation of hydrogel

A fibrous atelocollagen hydrogel (2.5 mg/mL atelocollagen, 0 µM cisplatin-DMSO complex, pH 7.4) and a platinum-crosslinked atelocollagen hydrogel crosslinked by a cisplatin-DMSO complex (2.5 mg/mL atelocollagen, 100 µM cisplatin-DMSO complex, pH 7.4) were incubated at 37°C for 30 minutes in a cell, and the resulting products were measured for transmittance of light with a wavelength of 200 to 900 nm using a spectrophotometer U5100 (HITACHI Corporation). The results are shown in FIG. 3.

### 3. Atelocollagen concentration evaluation (1)

Atelocollagen (final concentration: 0.1 to 2.5 mg/mL) and a 10 mM cisplatin-DMSO complex solution (final concentration: 100 µM) were mixed, and an atelocollagen/cisplatin-DMSO complex mixture solution containing a physiological phosphate buffer solution [30 mM phosphate buffer solution (pH 7.4), 120 mM NaCl] was prepared and incubated at 37°C for 30 minutes. Whether a hydrogel was formed or not was evaluated using whether the stainless steel ball was held on the interface or not as an indicator. As a control, an atelocollagen (final concentration: 0.1 to 2.5 mg/mL) solution was prepared as in above except that the cisplatin-DMSO complex solution was not contained, and the hydrogel-forming ability of the fibrous atelocollagen was evaluated. The results are shown in FIG. 4.

### 4. pH evaluation (1)

Atelocollagen (final concentration: 2.5 mg/mL) and a 10 mM cisplatin-DMSO complex solution (final concentration: 100 µM) were mixed, and atelocollagen/cisplatin-DMSO complex mixture solutions containing buffer solutions [30 mM buffer solution, 120 mM NaCl] with various pH values were prepared and incubated at 37°C for 30 minutes. As the 30 mM buffer solution, citrate buffer solutions were used for pH 4.0 and pH 5.0, phosphate buffer solutions were used for pH 6.0, pH 7.4, and pH 8.0, and a borate buffer solution was used for pH 9.0. Whether a hydrogel was formed or not was evaluated using whether the stainless steel ball was held on the interface or not as an indicator. As a control, the fibrous atelocollagen hydrogel-forming ability was evaluated by varying the pH value as in above except that the cisplatin-DMSO complex solution was not contained. The results are shown in FIG. 5.

### 5. Ionic strength evaluation (1)

Atelocollagen (final concentration: 2.5 mg/mL) and a 10 mM cisplatin-DMSO complex solution (final concentration: 100 µM) were mixed, and an atelocollagen/cisplatin-DMSO complex mixture solutions containing a 30 mM phosphate buffer solution (pH 7.4) and NaCl of various concentrations were prepared and incubated at 37°C for 30 minutes. The concentrations of NaCl were 0 mM, 70 mM, 120 mM, and 170 mM. Whether a hydrogel was formed or not was evaluated using whether the stainless steel ball was held on the interface or not as an indicator. The results are shown in FIG. 6.

### 6. Gelation time evaluation (1)

Atelocollagen (final concentration: 2.5 mg/mL) and a 10 mM cisplatin-DMSO complex solution (final concentration: 100 µM) were mixed, and an atelocollagen/cisplatin-DMSO complex mixture solution containing a physiological phosphate buffer solution [30 mM phosphate buffer solution (pH 7.4), 120 mM NaCl] was prepared and incubated at 37°C for 5 minutes, 10 minutes, 15 minutes, or 20 minutes. Whether a hydrogel was formed or not was evaluated using whether the stainless steel ball was held on the interface or not as an indicator. The results are shown in FIG. 7.

### 7. Gelation temperature evaluation (1)

Atelocollagen (final concentration: 2.5 mg/mL) and a 10 mM cisplatin-DMSO complex solution (final concentration: 100 µM) were mixed, and an atelocollagen/cisplatin-DMSO complex mixture solution containing a physiological phosphate buffer solution [30 mM phosphate buffer solution (pH 7.4), 120 mM NaCl] was prepared and incubated at 4°C, 20°C, or 37°C for 30 minutes. Whether a hydrogel was formed or not was evaluated using whether the stainless steel ball was held on the interface or not as an indicator. The results are shown in FIG. 8.

### 8. Study on method for removing free platinum complex by dialysis

An atelocollagen acidic solution (final concentration: 4.9 mg/mL) and a 10 mM cisplatin-DMSO complex solution (final concentration: 100 µM) were mixed and left to stand at 4°C for 3 days. This atelocollagen/cisplatin-DMSO complex mixture solution was transferred in a dialysis tube (fractional molecular weight: 12,000 to 14,000) and was immersed in a 1 mM HCl aqueous solution and left to stand at 4°C overnight. This atelocollagen/cisplatin-DMSO complex mixture solution (final concentration: 2.5 mg/mL) and a 1 mM HCl aqueous solution or 10× physiological phosphate buffer solution [final concentration: 30 mM phosphate buffer solution (pH 7.4), final concentration: 120 mM NaCl] were mixed and incubated at 37°C for 30 minutes. Whether a hydrogel was formed or not was evaluated using whether the stainless steel ball was held on the interface or not as an indicator. The results are shown in FIG. 9.

### 9. Gelation ability evaluation (2)

Atelocollagen (final concentration: 2.5 mg/mL) and a transplatin-DMSO complex solution of various concentrations (final concentration: 0 to 200 µM) were mixed, and an atelocollagen/transplatin-DMSO complex mixture solution containing a physiological phosphate buffer solution [30 mM phosphate buffer solution (pH 7.4), 120 mM NaCl] was prepared and incubated at 37°C for 30 minutes. Whether a hydrogel was formed or not was evaluated using whether the stainless steel ball was held on the interface or not as an indicator. The results are shown in FIG. 10.

### 10. Atelocollagen concentration evaluation (2)

Atelocollagen (final concentration: 0.1 to 2.5 mg/mL) and a 10 mM transplatin-DMSO complex solution (final concentration: 100 µM) were mixed, and an atelocollagen/transplatin-DMSO complex mixture solution containing a physiological phosphate buffer solution [30 mM phosphate buffer solution (pH 7.4), 120 mM NaCl] was prepared and incubated at 37°C for 30 minutes. Whether a hydrogel was formed or not was evaluated using whether the stainless steel ball was held on the interface or not as an indicator. The results are shown in FIG. 11.

### 11. pH evaluation (2)

Atelocollagen (final concentration: 2.5 mg/mL) and a 10 mM transplatin-DMSO complex solution (final concentration: 100 µM) were mixed, and atelocollagen/transplatin-DMSO complex mixture solutions containing physiological buffer solutions [30 mM buffer solution, 120 mM NaCl] with various pH values were prepared and incubated at 37°C for 30 minutes. Whether a hydrogel was formed or not was evaluated using whether the stainless steel ball was held on the interface or not as an indicator. As the 30 mM buffer solution, citrate buffer solutions were used for pH 4.0 and pH 5.0, phosphate buffer solutions were used for pH 6.0, pH 7.4, and pH 8.0, and a borate buffer solution was used for pH 9.0. The results are shown in FIG. 12.

### 12. Ionic strength evaluation (2)

Atelocollagen (final concentration: 2.5 mg/mL) and a 10 mM transplatin-DMSO complex solution (final concentration: 100 µM) were mixed, and atelocollagen/transplatin-DMSO complex mixture solutions containing a 30 mM phosphate buffer solution (pH 7.4) and NaCl of various concentrations were prepared and incubated at 37°C for 30 minutes. NaCl was used at 20 mM, 70 mM, 120 mM, or 170 mM. Whether a hydrogel was formed or not was evaluated using whether the stainless steel ball was held on the interface or not as an indicator. The results are shown in FIG. 13.

### 13. Gelation time evaluation (2)

Atelocollagen (final concentration: 2.5 mg/mL) and a 10 mM transplatin-DMSO complex solution (final concentration: 100 µM) were mixed, and an atelocollagen/transplatin-DMSO complex mixture solution containing a physiological phosphate buffer solution [30 mM phosphate buffer solution (pH 7.4), 120 mM NaCl] was prepared and incubated at 37°C for 5 minutes, 10 minutes, or 15 minutes. Whether a hydrogel was formed or not was evaluated using whether the stainless steel ball was held on the interface or not as an indicator. The results are shown in FIG. 14.

### 14. Gelation temperature evaluation (2)

Atelocollagen (final concentration: 2.5 mg/mL) and a 10 mM transplatin-DMSO complex solution (final concentration: 100 µM) were mixed, and an atelocollagen/transplatin-DMSO complex mixture solution containing a physiological phosphate buffer solution [30 mM phosphate buffer solution (pH 7.4), 120 mM NaCl] was prepared and incubated at 4°C, 24°C, or 37°C for 30 minutes. Whether a hydrogel was formed or not was evaluated using whether the stainless steel ball was held on the interface or not as an indicator. The results are shown in FIG. 15.

### 15. Evaluation of heat stability of hydrogel

A fibrous atelocollagen hydrogel (0 µM cisplatin-DMSO complex) of a final concentration of 2.5 mg/mL and an atelocollagen hydrogel crosslinked by a cisplatin-DMSO complex of a final concentration of 2.5 mg/mL (100 µM cisplatin-DMSO complex) were produced. The produced atelocollagen hydrogels were heated at 50°C, 60°C, or 70°C for 5 minutes each, and whether the stainless steel ball was held on the interface or not was checked. The results are shown in FIG. 16.

### 16. Study on heat shrinkability of hydrogel

An atelocollagen hydrogel (100 µM cisplatin-DMSO complex) crosslinked by a cisplatin-DMSO complex of a final concentration of 2.5 mg/mL was produced in a silicone support with a diameter of 10 mm and a thickness of 5 mm. The produced platinum-crosslinked atelocollagen hydrogel was poured with hot water of 80°C and immersed therein for 30 minutes. The results are shown in FIG. 17.

### 17. Evaluation of cytotoxicity and cell proliferation activity

A 4.0 mg/mL fibrous atelocollagen hydrogel and a 4.0 mg/mL platinum-crosslinked atelocollagen hydrogel crosslinked by a cisplatin-DMSO complex were produced in a culture dish with a diameter of 35 mm. HT-1080 cells were seeded on the produced fibrous atelocollagen hydrogel and platinum-crosslinked atelocollagen hydrogel at 8×10⁴ cells per dish and were cultured in DMEM containing 10% FBS at 37°C. After culturing for 1.5 hours to 67 hours, the cells were stained with Hoechst 33342 and calcein-AM for 30 minutes and were observed through a fluorescence microscope. The phase contrast microscopic images and fluorescence microscopic images at each time are shown in FIG. 18.

The number of cells per a visual field shown in FIG. 18 was counted. The numbers of cells in randomly selected 5 visual fields were counted and averaged. The results are shown in FIG. 19.

### 18. Cell infiltration evaluation

A 4 mg/mL fibrous atelocollagen hydrogel and a 4 mg/mL platinum-crosslinked atelocollagen hydrogel crosslinked by a cisplatin-DMSO complex were produced in a 24-well plate transwell insert. HT-1080 cells were seeded on the produced fibrous atelocollagen hydrogel and platinum-crosslinked atelocollagen gel at 3×10⁴ cells per well and were cultured in DMEM containing 10% FBS at 37°C for 4 days. On the second day of culturing, the medium was replaced by fresh one. After culturing for 4 days, the cells were fixed with 4% paraformaldehyde, and the hydrogel was paraffin-embedded and sliced. The produced slices were stained with hematoxylin and eosin (HE) and observed through a microscope. The results are shown in FIG. 20.

### II. Results

### 1. Gelation ability evaluation (1)

The atelocollagen/cisplatin-DMSO complex mixture solutions, excluding the solution in which the cisplatin-DMSO concentration was 25 µM, gelated at 37°C and held the stainless steel ball on their interfaces. The transparency of the collagen hydrogel increased with an increase in the cisplatin-DMSO complex concentration, and cloudiness was not almost observed at concentrations of 50 µM or more (FIG. 2).

### 2. Evaluation of transparency of hydrogel

The platinum-crosslinked atelocollagen hydrogel crosslinked by a cisplatin-DMSO complex exhibited high permeability in the visible light region (transparency almost the same as that of transparent plastic such as polystyrene) compared to a fibrous atelocollagen hydrogel (FIG. 3). This result suggests that although formation of atelocollagen fibers is prevented by adding a cisplatin-DMSO complex to atelocollagen, atelocollagen obtained polymer physical properties by being crosslinked (FIG. 1).

### 3. Atelocollagen concentration evaluation (1)

Gelation of the fibrous atelocollagen was observed at concentrations of 1.5 mg/mL or more, and the hydrogel was cloudy (FIG. 4). Gelation of the platinum-crosslinked atelocollagen crosslinked by a cisplatin-DMSO complex was observed at concentrations of 1.0 mg/mL or more, and the hydrogel was transparent (FIG. 4).

### 4. pH evaluation (1)

Gelation of the fibrous atelocollagen was observed at pH levels of 6.0 or more, and the hydrogel was cloudy (FIG. 5).

Gelation of the platinum-crosslinked atelocollagen crosslinked by a cisplatin-DMSO complex was observed at pH levels of 5.0 or more, and the hydrogel exhibited high transparency (FIG. 5), but cloudiness was observed at a pH of 9.0.

### 5. Ionic strength evaluation (1)

Gelation of the platinum-crosslinked atelocollagen crosslinked by a cisplatin-DMSO complex was observed in all solutions of the phosphate buffer solution of 30 mM and NaCl of 0 mM, 70 mM, 120 mM, and 170 mM, that is, at all concentrations of 50 mM, 100 mM, 150 mM, and 200 mM as the total ionic strength (FIG. 6). However, in 0 mM and 70 mM NaCl, the hydrogel became clouded.

### 6. Gelation time evaluation (1)

Gelation of the platinum-crosslinked atelocollagen crosslinked by a cisplatin-DMSO complex was observed after incubation at 37°C for 15 minutes, and the hydrogel exhibited high transparency (FIG. 7).

### 7. Gelation temperature evaluation (1)

Gelation of the platinum-crosslinked atelocollagen crosslinked by a cisplatin-DMSO complex was observed after incubation at all temperatures of 4°C, 20°C, and 37°C for 30 minutes, and the hydrogel exhibited high transparency (FIG. 8).

### 8. Study on method for removing free platinum complex by dialysis

The platinum-crosslinked atelocollagen crosslinked by a cisplatin-DMSO complex did not gelate in a 1 mM HCl aqueous solution but gelated at 37°C 30 minutes after changing the pH to 7.4, and the hydrogel exhibited high transparency (FIG. 9).

### 9. Gelation ability evaluation (2)

The atelocollagen/transplatin-DMSO complex mixture solutions gelated at every transplatin-DMSO concentration excluding 10 µM and 25 µM at 37°C, and held the stainless steel ball on the interface thereof (FIG. 10). At the transplatin-DMSO concentration of 25 µM, the gelation was moderate, and the stainless steel ball was held near the center. The transparency of the collagen hydrogel was enhanced with an increase in the transplatin-DMSO complex concentration, and almost no cloudiness was observed at 25 µM or more (FIG. 10).

### 10. Atelocollagen concentration evaluation (2)

Gelation of the platinum-crosslinked atelocollagen crosslinked by a transplatin-DMSO complex was observed at concentrations of 1.5 mg/mL or more, and the hydrogel exhibited high transparency (FIG. 11).

### 11. pH evaluation (2)

Gelation of the platinum-crosslinked atelocollagen crosslinked by a transplatin-DMSO complex was observed at pH levels of 6.0 or more, and the hydrogel exhibited high transparency (FIG. 12). However, cloudiness was observed at a pH of 9.0.

### 12. Ionic strength evaluation (2)

Gelation of the platinum-crosslinked atelocollagen crosslinked by a transplatin-DMSO complex was observed in the phosphate buffer solution of 30 mM and NaCl of all of 20 mM, 70 mM, 120 mM, and 170 mM, that is, at all concentrations of 50 mM, 100 mM, 150 mM, and 200 mM as the total ionic strength (FIG. 13). However, in 20 mM NaCl, the hydrogel became clouded.

### 13. Gelation time evaluation (2)

Gelation of the platinum-crosslinked atelocollagen crosslinked by a transplatin-DMSO complex was observed after incubation at 37°C for 15 minutes, and the hydrogel exhibited high transparency (FIG. 14).

### 14. Gelation temperature evaluation (2)

Hydrogelation of the platinum-crosslinked atelocollagen crosslinked by a transplatin-DMSO complex was observed after incubation at 24°C and 37°C for 30 minutes, and the hydrogel exhibited high transparency (FIG. 15), but gelation was not observed at 4°C for 30 minutes (FIG. 15).

### 15. Evaluation on heat stability of hydrogel

The fibrous atelocollagen hydrogel (0 µM cisplatin-DMSO complex) was fused by heating at 50°C for 5 minutes (FIG. 16).

The platinum-crosslinked atelocollagen hydrogel crosslinked by a cisplatin-DMSO complex (100 µM cisplatin-DMSO complex) shrank by heating at 70°C for 5 minutes, but was not completely fused (FIG. 16).

### 16. Evaluation of heat shrinkability of hydrogel

The fibrous atelocollagen hydrogel (0 µM cisplatin-DMSO complex) was fused immediately after being immersed in hot water of 80°C, and was diffused in water (FIG. 17; the photograph after immersion in hot water is not shown). This result suggests that the fibrous atelocollagen hydrogel was not crosslinked.

The platinum-crosslinked atelocollagen hydrogel crosslinked by a cisplatin-DMSO complex (100 µM cisplatin-DMSO complex) shrank when immersed in hot water of 80°C (FIG. 17). This result suggests the platinum-crosslinked atelocollagen hydrogel was crosslinked.

### 17. Cytotoxicity evaluation

Almost no dead cells were observed in both the fibrous atelocollagen hydrogel and the platinum-crosslinked atelocollagen hydrogel crosslinked by a cisplatin-DMSO complex (FIG. 18). In comparison of the shapes of the cells after culturing for 1.5 hours, a plurality of thin protrusions were formed in the fibrous atelocollagen hydrogel. In contrast, in the platinum-crosslinked atelocollagen hydrogel crosslinked by a cisplatin-DMSO complex, cells were stretched and stress fibers were formed (not shown).

Furthermore, the cell proliferation activity on the platinum-crosslinked atelocollagen hydrogel crosslinked by a cisplatin-DMSO complex was about 2 times higher than that on the fibrous hydrogel (FIG. 19).

### 18. Cell infiltration evaluation

Infiltration of cells were observed in both the fibrous atelocollagen hydrogel and the platinum-crosslinked atelocollagen hydrogel crosslinked by a cisplatin-DMSO complex, and it was suggested that the platinum-crosslinked atelocollagen hydrogel has biocompatibility equal to or higher than that of the fibrous atelocollagen hydrogel (FIG. 20).

### III. Evaluation as 3-dimensional cell culture substrate

Subsequently, the usefulness of the hydrogel composed of collagen crosslinked by a cisplatin-DMSO complex as a 3-dimensional cell culture substrate was evaluated.

### 1. Experimental method

Subconfluent MDCK cells derived from a canine renal tubular cell line were collected and resuspended in D-MEM containing 10% (v/v) FBS. A collagen acidic solution, a cisplatin-DMSO complex, and the cell suspension were mixed to give final concentrations of 4 mg/mL collagen, 100 µm cisplatin-DMSO complex, and 4×10³ cells/mL. This mixture was added to a 35-mm glass base dish and was incubated and gelated at 37°C for 45 minutes. Subsequently, 2 mL of a culture medium was added to the dish, and the medium was replaced by fresh one every 2 days. After culturing for 6 days, the cells were fixed in a 4% (v/v) p-formaldehyde/phosphate buffer solution for 15 minutes, permeabilized in 0.1% (v/v) Triton X-100/PBS for 15 minutes, and then blocked in 1% (w/v) BSA/PBS for 30 minutes. Nuclei and F-actin were stained with Hoechst 33342 and Phalloidin-iFluor 488 (AT Bioquest, Inc., Pleasanton, CA), respectively, for 3 hours, and fluorescence images were obtained using Fluoview FV-1000, a confocal fluorescence microscope (Olympus Corporation). The fluorescence intensities of five different nuclei in a cross-sectional image of a cell cluster were digitized using ImageJ (version 1.52a) and were plotted with respect to the depth from the bottom of the gel, which was compared to the fibrous atelocollagen hydrogel as a control.

### 2. Results

FIG. 21 shows fluorescence images of MDCK cell clusters in the platinum-crosslinked atelocollagen hydrogel crosslinked by a cisplatin-DMSO complex (hereinafter, referred to as "platinum(II) complex-crosslinked gel" or "PCG"). The MDCK cells were embedded in a 4 mg/mL fibrous atelocollagen hydrogel (FIG. 21A; hereinafter, referred to as "fibrous gel" or "FG") or PCG (FIG. 21B) and were cultured for 6 days, and the cells were then fixed and stained with Hoechst 33342 and Phalloidin-iFluor 488. The cells were observed using a confocal fluorescence microscope. The stains of blue and green indicate nuclei and F-actin, respectively. The depths of cell clusters from the bottom of the dish are shown in the images. The scale bar indicates 50 µm.

In the fibrous gel, it was difficult to obtain fluorescence images of F-actin and nuclei at 739 µm in the depth direction, but in the gel of the present invention in the right photograph, good fluorescence images could be obtained even at 803 µm.

FIG. 21C shows a relationship between cell depth and fluorescence intensity obtained by measuring fluorescence intensities of nuclei in cell clusters and plotting the intensities with respect to depth. Each plot shows mean ± standard deviation (n = 5).

In the gel of the present invention, a decrease in the fluorescence intensity with increasing depth was less compared to ordinary gels, and it was clarified that visual recognition thereof was possible.

### IV. Method for dissolving gel

### 1. Experimental method

Two hundred microliters of a 2.5 mg/mL atelocollagen gel containing 100 µM of a cisplatin-DMSO complex was prepared in a 1.5 mL tube, 200 µL of a 0.4 M reduced glutathione/PBS was added thereto, and the mixture was left to stand at room temperature. The dissolution of the gel was investigated by placing the stainless steel ball on the gel from above before addition of the glutathione solution and at 3 hours and 24 hours after addition of the glutathione solution.

### 2. Results

The results are shown in FIG. 22. The arrowheads indicate the positions of the stainless steel ball. The stainless steel ball was held on the surface of the hydrogel before the addition of the glutathione solution, but the stainless steel ball was sinking 3 hours after the addition of the glutathione solution by dissolution of the hydrogel and completely sank 24 hours after the addition of the glutathione solution.

That is, it was demonstrated that the hydrogel of the present invention was decrosslinked by addition of a thiol compound, reduced glutathione, and can be dissolved. This result demonstrates a characteristic that is obviously different from other crosslinking methods, and it is possible to collect spheroids cultured in the gel and to remove gels produced in vivo, depending on the situation.

### V. Method for adjusting gel hardness (1)

### 1. Experimental method

Silicone sheets having a thickness of 5 mm and provided with a 10 mm diameter hole were placed on Parafilm, and 4 mg/mL atelocollagen gels containing transplatin-DMSO of various concentrations were produced in the holes. The silicone sheets were removed, and the gels were pressed down from above with a spatula.

### 2. Results

The results are shown in FIG. 23. When the addition amount of the transplatin-DMSO complex was 50 µM, the gel trembled when shaken, deformed by pressing down from above, and was quite sticky. The result, when the addition amount of the transplatin-DMSO complex was 100 µM, demonstrated that although the gel almost did not tremble when shaken, the gel deformed by pressing down from above but returned. The result when the addition amount of the transplatin-DMSO complex was 300 µM demonstrated that although the gel did not tremble when shaken, the gel slightly deformed by pressing down from above and returned. The result when the addition amount of the transplatin-DMSO complex was 500 µM demonstrated that although the gel did not tremble when shaken, the gel slightly deformed by pressing down from above and returned. The result when the addition amount of the transplatin-DMSO complex was 1000 µM demonstrated that although the gel did not tremble when shaken, the gel slightly deformed by pressing down from above and returned. The gels deformed in a way that was less influenced by the concentration of the transplatin-DMSO complex, yet the repulsive force increased proportionally. That is, it was suggested that the hardness of the hydrogel of the present invention can be adjusted by adjusting the concentration of the transplatin-DMSO complex.

### VI. Method for adjusting gel hardness (2)

### 1. Experimental method

In a 24-well plate, 4.0 mg/mL atelocollagen gels containing a transplatin-DMSO complex of 0, 50, 100, 200, 400, or 800 µM were prepared, and the stress when a cylinder with a diameter of 8 mm was pushed into the gel at a speed of 1 mm/min was measured with a universal tester (UCT-500, A&D Co., Ltd.). The slope of the stress-strain curve in the elastic region was calculated as the elastic modulus. Each bar shows mean ± standard deviation (n = 3).

### 2. Results

The results are shown in FIG. 24. When the addition amount of the transplatin-DMSO complex was 50 µM to 400 µM, the elastic modulus of the gel increased with the concentration of the complex and reached the plateau at 400 µM or more. That is, it was demonstrated that within a range of the transplatin-DMSO complex concentration of 400 µM or less, the hardness of the gel can be adjusted.

### VII. Summarization

From the above results, the hydrogel of the present invention provides characteristics in which:
- the transparency is high;
- gel formation can be controlled by pH;
- the gel strength can be controlled by the concentration of a platinum(II) complex;
- gelation is reversible and the gel can be dissolved; and
- the unreacted crosslinking agent can be removed.

Consequently, compared to gels composed of known collagen, the following advantages can be achieved:
(1) cell observation is easy and good data can be efficiently obtained even in a thick 3D culture;
(2) after being injected into a living body, such as the eyeball, and undergoing gelation, the gel can be dissolved and removed;
(3) the hardness of the gel can be adjusted according to the purpose, and it is therefore expected to be able to adjust the biodegradability; and
(4) the unreacted crosslinking agent does not cause any toxicity.

### Industrial Applicability

The hydrogel of the present invention can be used as a material for medicine, such as a material for eye disease treatment, an artificial vitreous body, a hemostatic agent (preferably a tamponade material), a sustained-release drug carrier, and a tissue replacement material, and as a material for research, such as a scaffold material for cell culture.

## Claims

1. A hydrogel comprising platinum-crosslinked collagen in which collagen is crosslinked by a platinum(II) complex, wherein the hydrogel exhibits a visible light transmittance of 60% or more when the transmittance is measured at an absorbance of 400 nm in a cell with a light path length of 10 mm.

2. The hydrogel comprising platinum-crosslinked collagen in which collagen is crosslinked by a platinum(II) complex according to claim 1, wherein the hydrogel exhibits a visible light transmittance of 60% or more when the transmittance is measured at an absorbance of 380 to 780 nm in a cell with a light path length of 10 mm.

3. The hydrogel according to claim 1, wherein the platinum(II) complex includes dimethyl sulfoxide (DMSO) as a ligand.

4. The hydrogel according to claim 3, wherein the platinum(II) complex is a complex in which at least one ligand of a chloride ion and/or an ammine of cis-diamminedichloroplatinum(II) (cisplatin) and/or trans-diamminedichloroplatinum(II) (transplatin) is substituted with DMSO.

5. The hydrogel according to claim 1, wherein the collagen is atelocollagen.

6. The hydrogel according to any one of claims 1 to 5, wherein the hydrogel is for use as an artificial vitreous body, a hemostatic agent, a sustained-release drug carrier, a tissue replacement material, or a scaffold material for cell culture.

7. A material for eye disease treatment, comprising the hydrogel according to any one of claims 1 to 5.

8. An artificial vitreous body comprising the hydrogel according to any one of claims 1 to 5.

9. A hemostatic agent comprising the hydrogel according to any one of claims 1 to 5.

10. A scaffold material for cell culture, comprising the hydrogel according to any one of claims 1 to 5.

11. A method for manufacturing the hydrogel according to any one of claims 1 to 5, comprising a step of mixing and incubating collagen and a platinum(II) complex.

12. The manufacturing method according to claim 11, comprising a step of incubating the collagen having a concentration in a range of 1.0 to 4.0 mg/mL and the platinum(II) complex having a concentration in a range of 25 µM to 1 mM under conditions of a pH in a range of 5.0 to 8.0.

13. A method for manufacturing the hydrogel according to any one of claims 1 to 5, comprising the steps of:
(a) incubating collagen having a concentration in a range of 1.0 to 4.0 mg/mL and a platinum(II) complex having a concentration in a range of 25 µM to 1 mM under conditions of a pH less than 5.0; and
(b) changing the pH condition to exhibit a pH of 5.0 to 8.0 to induce gelation.

14. The manufacturing method according to claim 13, comprising:
between the steps (a) and (b),
(a') a step of performing dialysis under conditions of a pH less than 5.0 to remove free platinum(II) complex.
